# EUROPEAN PATENT APPLICATION

(11) **EP 4 752 120 A1**
(43) Date of publication of application: **03.06.2026**
(21) Application number: 25214780.6
(22) Date of filing: 11.11.2025
(51) Int. Cl.: C04B 35/486, A61K 6/818

(54) **DENTAL COMPOSITION AND PROSTHESIS USING SAME**

(30) Priority: 26.11.2024 KR 20240171368
(71) Applicant: Vatech mcis co., Ltd, Suwon-si, Gyeonggi-do (KR); Vatech Ewoo Holdings Co., Ltd., Hwaseong-si, Gyeonggi-do, 18449 (KR)
(72) Inventor: KIM, Dae Jun, 18449 Hwaseong-si (KR); YANG, Gi Uk, 18449 Hwaseong-si (KR); HONG, Ju Seop, 18449 Hwaseong-si (KR)
(74) Representative: Lorenz & Kollegen

(57) **Abstract**

Proposed is a dental composition. The dental composition includes 20 to 40wt% of dispersed phase zirconia solid solution and 60 to 80 wt% of matrix phase zirconia solid solution.

## Description

### CROSS REFERENCE TO RELATED APPLICATION

The present application claims priority to Korean Patent Application No. 10-2024-0171368, filed November 26, 2024, the entire contents of which are incorporated herein for all purposes by this reference.

### BACKGROUND

### Technical Field

The present disclosure relates to a dental composition and a prosthesis using the same.

### Description of the Related Art

Around the same time that titanium implants were developed in the 1960s, the search for alternative materials for titanium dental implants began. This search was driven by the fact that approximately 0.6% of implant patients exhibit allergic reactions to titanium, and may be considered as part of the solution to the implant surface oxidation, even though histological inflammation does not occur.

Recently, due to high patient demand for aesthetic dental treatments, there has been an increasing need to overcome complaints about the gray color of titanium metal showing dark through the surrounding tissue, especially in cases of thin gum tissue.

Furthermore, as clinical evidence continues to link titanium to mucositis caused by chain chemical reactions within the oral cavity, the development and commercialization of ceramic implants which can resolve these issues is underway.

The ceramic implant material originated with presentation of alumina (Al₂O₃) in 1962. Sapphire, a single crystal of alumina with improved physical properties, was first used clinically in Japan in 1972 and introduced to the United States in 1980. However, due to the low fracture toughness, sapphire has a high fracture rate of approximately 35% in clinical uses over the long term.

Tetragonal zirconia (ZrO₂) implants, which are stable and overcome the mechanical weakness of alumina due to the addition of yttria (Y₂O₃), were first marketed in 1985 and are still use today. Recently, zirconia implants have been used.

### SUMMARY

An embodiment of the disclosure provides a zirconia dental material which may overcome low-temperature degradation and enhance resistance to defects to compensate for the disadvantages of zirconia such as low-temperature degradation and brittle fracture.

An embodiment of the disclosure provides a zirconia dental material provides a zirconia dental material which is resistant to defects through composite formation using a matrix phase zirconia solid solution for stabilization and a dispersive phase zirconia solid solution for destabilization.

An embodiment of the disclosure provides a zirconia dental material, to which Nb⁵⁺ having a smaller ionic radius and a higher oxidation state than Zr⁴⁺ is added, to reduce the creation of oxygen vacancies known to be associated with low-temperature degradation.

An embodiment of the disclosure provides a zirconia dental material which enhances resistance to defects by inducing appropriate destabilization of the tetragonal phase and promoting phase transformation into the monoclinic phase.

According to embodiments, a dental composition includes 20 to 40wt% of dispersed phase zirconia solid solution; and 60 to 80 wt% of matrix phase zirconia solid solution.

According to an embodiment, the dispersed phase zirconia solid solution may include 94.5 to 96.5 mol% of ZrO₂, 2.5 to 3.5 mol% of Y₂O₃, and 1.0 to 2.0 mol% of Nb₂O₅.

According to an embodiment, the matrix phase zirconia solid solution may include 90 to 92 mol% of ZrO₂, 4.5 to 5.5 mol% of Y₂O₃, and 3.5 to 4.5 mol% of Nb₂O₅.

According to an embodiment, the dispersed phase zirconia solid solution may have a grain size of 0.1 to 0.4 µm.

According to embodiments, a prosthesis includes the dental composition described above.

According to an embodiment, low-temperature degradation may be overcome and resistance to defects may be enhanced to compensate for the disadvantages of zirconia such as low-temperature degradation and brittle fracture.

According to an embodiment, a zirconia dental material resistant to defects is provided through composite formation using a matrix phase zirconia solid solution for stabilization and a dispersive phase zirconia solid solution for destabilization.

According to an embodiment, by adding Nb⁵⁺ having a smaller ionic radius and a higher oxidation state than Zr⁴⁺ to reduce the creation of oxygen vacancies known to be associated with low-temperature degradation, low-temperature degradation is overcome.

According to an embodiment, resistance to defects is enhanced by inducing appropriate destabilization of the tetragonal phase and promoting phase transformation into the monoclinic phase.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a graph illustrating changes in biaxial strength after indentation of a dental composition of the disclosure and a conventional material;
FIG. 2 illustrates captured images of crack lengths in the dental composition of the disclosure and the conventional material after 490 N indentation;
FIG. 3 illustrates the comparison of XRD diffraction changes before and after low-temperature degradation between the dental composition of the disclosure and the conventional material;
FIG. 4 illustrates the comparison of biaxial strength changes before and after low-temperature degradation between the dental composition of the disclosure and a conventional material;
FIG. 5 illustrates the comparison of crystal structure images between the dental composition of the disclosure and a conventional material; and
FIG. 6 is a perspective view illustrating an implant structure of the dental composition of the disclosure.

### DETAILED DESCRIPTION

Hereinafter, implementations of the disclosure will be described in detail. However, these implementations are presented merely as examples, by which the disclosure is not limited. The disclosure is defined solely by the scope of the claims set forth below.

Unless otherwise specified herein, when a portion such as a layer, a film, a region, or a plate is described as being "on" another portion, it should be understood that the portion may be "directly on" the other portion or indirectly on the other portion via an intervening portion.

Unless otherwise specified herein, singular forms may include plural forms. Unless otherwise specified, "A or B" may refer to "including A, including B, or including A and B."

As used herein, the phrase "combination thereof" may refer to, for example, a mixture, a stack, a composite, a copolymer, an alloy, a blend, or a reaction product of constituents.

Methods and materials similar or equivalent to those described herein may be used in the practice or testing of the disclosure, but suitable methods and materials are described herein. Unless clearly stated otherwise in the context, singular forms include plural forms.

It will be understood herein that the terms "comprise", "include", "have", etc. when used in the specification, specify the presence of stated features, numbers, steps, operations, elements, parts, components, materials, and/or combinations thereof but do not preclude the presence or addition of one or more other features, integers, steps, operations, elements, parts, components, materials, and/or combinations thereof.

As used herein, the term "a combination thereof" indicates a mixture or combination of two or more components described. The combination thereof may refer to, for example, a mixture, a stack, a composite, a copolymer, an alloy, a blend, or a reaction product of constituents.

As used herein, the term "and/or" refers to including any and all combinations of one or more of the items described in relation thereto. The term "or" in this specification refers to "and/or".

Hereinafter, example embodiments will be described in more detail.

### Zirconia Solid Solution with Nb⁵⁺ Added

According to an embodiment of the disclosure, a process for adding Nb⁵⁺ is as follows: Zr, Y, and Nb are mixed in distilled water via ball milling for 12 to 48 hours, depending on the content of the base and dispersed phases. Thereafter, the mixed slurry is dried and powdered, and then is subjected to solution treatment at a temperature of 1,000 to 1,300°C for 5 to 10 hours to produce base-phase powder and dispersed phase powder.

The dental composition according to the disclosure includes a dispersed phase zirconia solid solution and a matrix phase zirconia solid solution at a ratio of 2:8 to 4:6, containing 20 to 40 wt% of the dispersed phase zirconia solid solution and 60 to 80 wt% of the matrix phase zirconia solid solution.

**[Table 1]**

| | | Dispersed Phase (20 to 40 wt%) | Matrix phase (60 to 80 wt%) |
|---|---|---|---|
| Composition Content | ZrO₂ | 94.5 to 96.5 mol% | 90 to 92 mol% |
| | Y₂O₃ | 2.5 to 3.5 mol% | 4.5 to 5.5 mol% |
| | Nb₂O₅ | 1.0 to 2.0 mol% | 3.5 to 4.5 mol% |

### Dispersed phase Zirconia

During the sintering process, the tetragonal phase of zirconia transforms into the monoclinic phase during cooling at high temperatures, thereby resulting in a volume expansion of approximately 3%. This expansion may create microcracks which may degrade material properties. According to an embodiment of the disclosure, to prevent this degradation, Y³⁺ is added to zirconia to stabilize zirconia so that zirconia maintains the tetragonal phase even at room temperature.

When cracks are created due to external forces, localized phase transformation into a monoclinic structure occurs. The resulting localized volume expansion counteracts the force that widens cracks, thereby preventing crack propagation and enhancing resistance to defects. This process is referred to as zirconia toughening. According to an embodiment of the disclosure, a dispersed powder was produced by adding Nb⁵⁺ to further promote the toughening mechanism and developing a composition with increased instability.

### Matrix phase Zirconia

Zirconia undergoes low-temperature degradation, which causes the tetragonal phase to transform into the monoclinic phase. This results in overall volume expansion, leading to the formation of microcracks and subsequent deterioration in material properties. This low-temperature degradation is known to be related to oxygen vacancies in zirconia. According to an embodiment of the disclosure, to suppress this phenomenon, Nb⁵⁺ is added to produce matrix phase powder which is stable at low temperatures.

### Defect Resistance Test

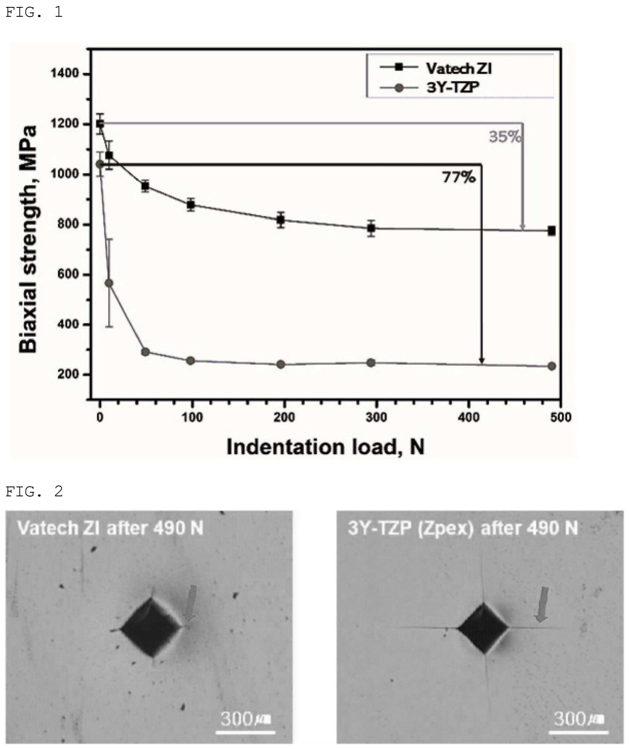

Changes in biaxial strength were compared between zirconia "Vatech ZI" according to an example of the disclosure and a conventional sintered material "3Y-TZP" (Tosoh Zpex^{®}) after indentation at different loads using a Vickers indenter. For the comparison results, refer to FIGS. 1 and 2.

FIG. 1 is a graph illustrating changes in biaxial strength after indentation of a dental composition of the disclosure and a conventional material, and FIG. 2 illustrates captured images of crack lengths in the dental composition of the disclosure and the conventional material after 490 N indentation.

After Vickers indentation at 490N, the reduction in biaxial strength for Vatech ZI was approximately 35%, while the reduction in biaxial strength for the conventional material was approximately 77%. The defect resistance of Vatech ZI according to an example of the disclosure was confirmed to be significantly superior.

### Low-Temperature Degradation Stability Test

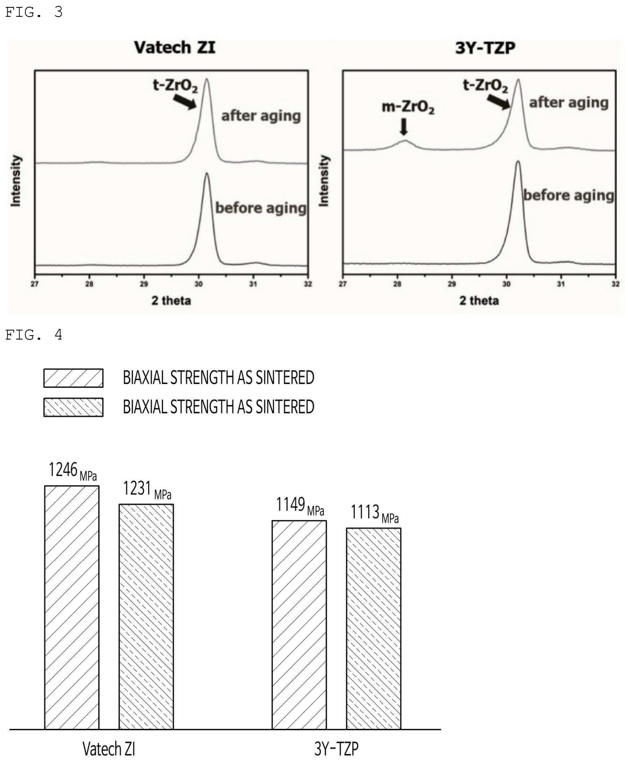

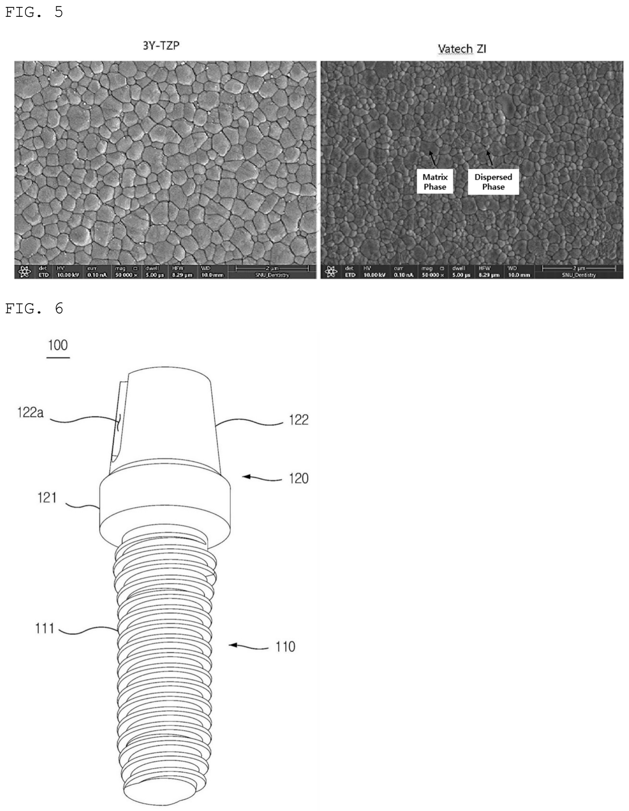

A 5-hour heat treatment was carried out under autoclave conditions of 134°C and 2 bar pressure, as specified by ISO 13356 for low-temperature degradation testing. FIG. 3 illustrates the comparison of XRD diffraction changes before and after low-temperature degradation between the dental composition of the disclosure and the conventional material. FIG. 4 illustrates the comparison of biaxial strength changes before and after low-temperature degradation between the dental composition of the disclosure and a conventional material. FIG. 5 illustrates the comparison of crystal structure images between the dental composition of the disclosure and a conventional material. XRD diffraction patterns before and after low-temperature aging showed no significant change in the dental composition according to the disclosure, but a slight increase in the monoclinic phase was observed for the conventional material. The composition table of the dental composition according to the disclosure used in the test is as follows.

**[Table 2]**

| | | Dispersed Phase (20 to 40 wt%) | Matrix phase (60 to 80 wt%) |
|---|---|---|---|
| Grain Size | | 0.1 to 0.4 µm | 0.5 to 1.5 µm |
| Composition Content | ZrO₂ | 94.5 to 96.5 mol% | 90 to 92 mol% |
| | Y₂O₃ | 2.5 to 3.5 mol% | 4.5 to 5.5 mol% |
| | Nb₂O₅ | 1.0 to 2.0 mol% | 3.5 to 4.5 mol% |

### Implant Structure

FIG. 6 is a perspective view illustrating an implant structure for a prosthesis, with the above-described dental material being used in the implant structure. Dental prosthetic compositions include the materials, as well as semi-finished products, such as disks and blocks, for fabricating prostheses. Prostheses comprehensively include any prosthetic structure such as crowns, inlays, and implant structures.

Implants require strong osseointegration and durability to be reliably fixed in the oral cavities of patients. According to an embodiment of the disclosure, the zirconia implant structure may include an upper structure and a lower structure. The upper structure and the lower structure may be integrally provided. The integrated upper and lower structures serve to support the prosthesis, thereby requiring different surface treatment techniques for respective regions.

Referring to FIG. 6, an implant structure 100 according to an embodiment of the disclosure may be configured to extend in a vertical direction.

The implant structure 100 may be configured such that one side is implanted into the alveolar bone where a tooth is missing, with a prosthesis fixedly fitted to the other side. Due to this configuration, the implant structure 100 may help restore the function of the missing tooth. The implant structure 100 may be formed from various materials. For example, the implant structure 100 may be formed from a zirconia material.

The implant structure 100 may include a lower structure 110. The lower structure 110 may be implanted into the alveolar bone as an artificial root. The lower structure 110 may be formed as a cylinder extending in the vertical direction. The lower structure 110 may have a threaded portion 111 formed on the outer circumference. The lower structure 110 may be engaged with and implanted into the alveolar bone through the threaded portion 111. The diameter of the lower structure 110 may progressively decrease in one direction. For example, the diameter of the lower structure 110 may progressively decrease from the upper side to the lower side.

The implant structure 100 may include an upper structure 120. The upper structure 120 may serve as an abutment for fixing the prosthesis. The upper structure 120 may be connected to one end of the lower structure 110. For example, the upper structure 120 may be connected directly and integrally to the upper end of the lower structure 110. Accordingly, the implant structure 100 may be formed as one piece including the lower structure 110 and the upper structure 120.

The upper structure 120 may include a gingival portion 121. The gingival portion 121 may be connected to the upper portion of the lower structure 110. The lower end of the gingival portion 121 may be integrally provided on the upper end of the lower structure 110. The gingival portion 121 may be provided as a cylinder having a larger diameter than the lower structure 110. The bottom surface and the top surface of the gingival portion 121 may each be formed as a tapered inclined surface.

The upper structure 120 may include a prosthesis support 122. The prosthesis support 122 may be positioned on the upper portion of the gingival portion 121. The lower end of the prosthesis support 122 may have a stepped portion integrally provided on the upper end of the gingival portion 121. The prosthesis support 122 may have a larger diameter than the lower structure 110 and a smaller diameter than the gingival portion 121. The prosthesis support 122 may be provided in the shape of a frustum. The outer peripheral surface of the prosthesis support 122 may be formed as a tapered inclined surface. The outer peripheral surface of the prosthesis support 122 may be inclined such that the upper end is narrower than the lower end, i.e., the diameter thereof gradually increases from the upper side to the lower side.

Because the implant structure 100 is configured as one piece, the lower structure 110, the gingival portion 121, and the prosthesis support 122 may be manufactured as a unitary structure.

The upper structure 120 may be provided with a rotation-limiting groove 122a. The rotation stop recess 122a may be provided on the outer peripheral surface of the upper structure 120. For example, the rotation stop recess 122a may be provided on the outer peripheral surface of the prosthesis support 122. The rotation stop recess 122a may be provided concavely inward from the outer surface of the prosthesis support 122. The rotation stop recess 122a may be formed elongated in the vertical direction. The rotation stop recess 122a may be inclined at the same inclination angle as the outer peripheral surface of the prosthesis support 122. The inner surface of the rotation stop recess 122a may be formed rounded. For example, the inner surface of the rotation stop recess 122a may be formed in an arc shape when viewed in cross-section.

Although the disclosure has been described in detail with reference to the example embodiments, the disclosure is not limited to these embodiments. Various modifications are possible within the scope of the claims, the detailed description of the disclosure, and the accompanying drawings, and such modifications should also fall within the scope of the disclosure.

## Claims

1. A dental composition comprising:
20 to 40wt% of dispersed phase zirconia solid solution; and
60 to 80 wt% of matrix phase zirconia solid solution.

2. The dental composition of claim 1, wherein the dispersed phase zirconia solid solution comprises 94.5 to 96.5 mol% of ZrO₂, 2.5 to 3.5 mol% of Y₂O₃, and 1.0 to 2.0 mol% of Nb₂O₅.

3. The dental composition of claim 1, wherein the matrix phase zirconia solid solution comprises 90 to 92 mol% of ZrO₂, 4.5 to 5.5 mol% of Y₂O₃, and 3.5 to 4.5 mol% of Nb₂O₅.

4. The dental composition of claim 1, wherein the dispersed phase zirconia solid solution has a grain size of 0.1 to 0.4 µm.

5. A prosthesis comprising a dental composition, the dental composition comprising:
20 to 40wt% of dispersed phase zirconia solid solution; and
60 to 80 wt% of matrix phase zirconia solid solution.
